**Europäisches Patentamt**

(19) **European Patent Office**

**Office européen des brevets**

(11) Numéro de publication: **0 006 791**
**B1**

(12) **FASCICULE DE BREVET EUROPEEN**

(45) Date de publication du fascicule du brevet: **29.12.82**　(51) Int. Cl.³: **C 07 C 39/12**

(21) Numéro de dépôt: **79400379.8**

(22) Date de dépôt: **12.06.79**

(54) **Nouveau procédé de préparation du tétrahydro-1,2,3,4 anthracène-diol-9,10.**

(30) Priorité: **29.06.78 FR 7819465**

(43) Date de publication de la demande:
**09.01.80 Bulletin 80/1**

(45) Mention de la délivrance du brevet:
**29.12.82 Bulletin 82/52**

(84) Etats contractants désignés:
**AT BE CH DE FR IT SE**

(56) Documents cités:
**néant**

(73) Titulaire: **P C U K PRODUITS CHIMIQUES UGINE KUHLMANN**
**Service Propriété Industrielle Tour Manhattan**
**F-92087 Paris La Defense 2 Cédex 21 (FR)**

(72) Inventeur: **Delavarenne, Serge**
**2, rue des Alouettes**
**F-69340 Francheville-le-Haut (FR)**
Inventeur: **Tellier, Pierre**
**106, Avenue de la Libération**
**69110 Sainte-Foy-Les-Lyon (FR)**

(74) Mandataire: **Houssin, Jean et al,**
**Produits Chimiques Ugine Kuhlmann Service**
**Propriété Industrielle Tour Manhattan Cedex 21**
**F-92087 Paris la Défense (FR)**

Courier Press, Leamington Spa, England.

## 0 006 791

### Nouveau procédé de préparation du tétrahydro-1,2,3,4 anthracène-diol-9,10

La présente invention concerne un nouveau procédé de préparation du tétrahydro-1,2,3,4 anthracène-diol-9,10.

On sait que le tétrahydro - 1,2,3,4 anthracène - diol - 9,10 peut être obtenu par hydrogénation catalytique de l'anthraquinone (SKITA—Ber. 1925 *58*—2685—97). Mais cette réaction très peu sélective ne permet pas d'obtenir de bons rendements en tétrahydro - 1,2,3,4 anthracène - diol - 9,10.

La demanderesse a maintenant trouvé qu'il était possible d'obtenir ce produit avec de très bons rendements. Le procédé selon l'invention pour la préparation du tétrahydro - 1,2,3,4 anthracène - diol - 9,10 est caractérisé en ce que l'on effectue l'hydrogénation catalytique en phase liquide de la tétrahydro - 1,4,4a,9a anthraquinone en hexahydro - 1,2,3,4,4a,9a anthracène - dione - 9,10 en présence d'un catalyseur à base de nickel ou de métal précieux, à une température comprise entre 20 et 200°C et effectue l'isomérisation de l'hexahydro - 1,2,3,4,4a,9a anthracène - dione - 9,10 en tétrahydro - 1,2,3,4 anthracène - diol - 9,10 en présence d'un acide en l'absence d'oxygène. Ces deux réactions peuvent être réalisées successivement ou simultanément. Dans ce dernier cas, l'hydrogénation est effectuée en présence d'un acide.

Les avantages du procédé de l'invention sont multiples: il fait appel à une matière première peu coûteuse, la tétrahydro - 1,4,4a,9a anthraquinone, accessible facilement par synthèse diénique à partir de naphtoquinone et de butadiène. L'hydrogénation peut être réalisée en présence de catalyseurs autres que ceux à base de métaux précieux: les catalyseurs à base de nickel conviennent parfaitement. Enfin la transformation de la tétrahydro - 1,4,4a,9a anthraquinone en tétrahydro - 1,2,3,4 anthracène - diol se fait avec un très bon rendement.

L'hydrogénation de la tétrahydro - 1,4,4a,9a anthraquinone selon l'invention conduit dans la plupart des cas à un mélange d'hexahydro - 1,2,3,4,4a,9a anthracène - dione - 9,10 et de tétrahydro - 1,2,3,4 anthracène - diol - 9,10 en proportions variables suivant les conditions de réaction. Il est possible de séparer ces deux composés par les moyens connus, en particulier en mettant à profit leur différence de solubilité, et de n'effectuer l'isomérisation que sur l'hexahydro - 1,2,3,4,4a,9a anthracène - dione - 9,10 ainsi isolé. Il est également possible, et c'est un des grands avantages de la présente invention, de soumettre au traitement d'isomérisation le mélange des deux composés. Une autre possibilité est d'effectuer l'hydrogénation dans un solvant qui entraine simultanément l'isomérisation de l'hexahydro - 1,2,3,4,4a,9a anthracène - dione - 9,10 en tétrahydro - 1,2,3,4 anthracène - diol - 9,10 de façon à obtenir directement ce dernier en une seule étape.

Selon le procédé de l'invention, l'hydrogénation est effectuée en phase liquide, la tétrahydro - 1,4,4a,9a anthraquinone étant en solution dans un des solvants couramment utilisés pour les hydrogénations. A titre d'exemples de tels solvants on peut citer les hydrocarbures aliphatiques, cycloaliphatiques ou aromatiques, les éthers, le tétrahydrofuranne, le dioxanne, les alcools. D'une manière générale, tous les solvants ne comportant pas un groupement fonctionnel hydrogénable dans les conditions de la réaction peuvent être utilisés.

Le catalyseur est choisi parmi ceux habituellement utilisés en hydrogénation, notamment ceux à base de nickel, comme le nickel de Raney, ou ceux à base de métaux précieux comme le palladium ou le platine.

La réaction d'hydrogénation est effectuée dans une large gamme de température allant de 20 à 200°C. On préfère opérer entre 60 et 130°C.

L'hydrogénation peut être réalisée à la pression atmosphérique mais elle est alors lente. Il est donc préférable d'opérer sous pression. Il n'y a pas de limite supérieure de la pression d'hydrogénation qui est choisie de préférence entre 10 et 50 bars. L'admission d'hydrogène est maintenue jusqu'à ce que la quantité théorique d'hydrogène correspondant à la formation de l'hexahydro - anthracène - dione ait été absorbée.

La concentration en tétrahydro - 1,4,4a,9a anthraquinone dans le milieu de réaction peut également varier dans de larges limites. Il n'y a pas de limite inférieure de cette concentration, mais pour des raisons de productivité elle est de préférence supérieure à 10% en poids. Pour des raisons pratiques, elle est inférieure à 50%. La concentration préférée est comprise entre 15 et 40%.

Selon le procédé de l'invention, l'isomérisation de l'hexahydro - 1,2,3,4,4a,9a anthracène - dione - 9,10 est effectuée par un traitement en milieu acide en l'absence d'oxygène. L'acide utilisé pour réaliser l'isomérisation peut être un acide minéral ou un acide organique. Comme exemple d'acide minéral on peut citer l'acide sulfurique, l'acide chlorhydrique, l'acide phosphorique. Les acides organiques sont constitués par les acides carboxyliques parmi lesquels on peut citer par exemple, l'acide formique, l'acide acétique, l'acide propionique, les acides butyriques.

L'isomérisation peut être effectuée à des températures aussi basses que 20°C mais elle est alors lente. Pour cette raison on préfère opérer à une température plus élevée choisie de préférence entre 50 et 200°C. Si l'on veut effectuer l'hydrogénation et l'isomérisation en une seule étape, on effectue l'hydrogénation de la tétrahydro - 1,4,4a,9a anthraquinone en présence d'un acide minéral ou organique tels que ceux mentionnés ci-dessus.

2

Les exemples qui suivent illustrent sans la limiter la présente invention.

Exemple 1

Dans un autoclave en acier inoxydable muni de dispositifs de chauffage et d'agitation on introduit 100 ml de toluène, 21,2 g de tétrahydro - 1,4,4a,9a anthraquinone et 0,2 g d'un catalyseur à base de palladium déposé sur charbon, contenant 5% de palladium. On chauffe à 100°C et introduit de l'hydrogène sous une pression de 30 bars. On poursuit la réaction pendant 4 heures en maintenant la pression entre 20 et 30 bars. Après refroidissement on sépare par filtration 7 g d'une première fraction constituée de tétrahydro - 1,2,3,4 anthracène - diol - 9,10 fondant à 206—208°C, caractérisé par ses spectres IR et de RMN. Par concentration du filtrat on obtient 14 g d'une deuxième fraction constituée par un produit fondant à 80—88°C dont les spectres de masse IR et RMN montrent qu'il s'agit d'hexahydro - 1,2,3,4,4a,9a anthracène - dione - 9,10 pratiquement pure. Cette deuxième fraction est mise en solution dans 42 g d'acide acétique et chauffée sous azote à la température d'ébullition du mélange pendant 4 heures. Après refroidissement à 0°C on filtre les cristaux qui se sont formés: on les lave à l'eau et on les sèche sous pression réduite. On recueille ainsi 12,7 g d'un produit cristallisé fondant à 210—212°C, dont le spectre IR est le même que celui de la première fraction, et qui est constitué par du tétrahydro - 1,2,3,4 anthracène - diol - 9,10. Le rendement global est de 92% sans prendre en compte la fraction de produit restant en solution dans l'acide acétique.

Exemple 2

On effectue l'hydrogénation de la tétrahydro - 1,4,4a,9a anthraquinone en opérant comme à l'exemple 1 mais en remplaçant le catalyseur sur palladium par 2 g d'un catalyseur au nickel obtenu par attaque à la soude de 4 g d'un alliage nickel aluminium à 50% de nickel, et en effectuant la réaction à 60°C pendant 5 heures 1/2 sous une pression d'hydrogène de 30 bars. On sépare le catalyseur et évapore le solvant sous pression réduite. On obtient 20,2 g d'un résidu que l'on traite avec 60 g d'acide acétique comme à l'exemple 1. On obtient finalement 18,4 g de tétrahydro - 1,2,3,4 anthracène - diol - 9,10 dont le spectre IR est le même que celui du produit obtenu à l'exemple 1. Le rendement est de 86% sans prendre en compte la fraction de produit restant en solution dans l'acide acétique.

Exemple 3

Dans un autoclave on introduit 300 ml d'acide acétique, 64 g de tétrahydro - 1,4,4a,9a anthraquinone et 0,6 g d'un catalyseur à 5% de palladium déposé sur charbon. On chauffe à 100°C et introduit de l'hydrogène sous une pression de 30 bars. On poursuit la réaction pendant 4 heures en maintenant la pression entre 20 et 30 bars. Après refroidissement on sépare par filtration le précipité obtenu, on le lave à l'eau et le sèche. On obtient 56 g de tétrahydro - 1,2,3,4 anthracène - diol - 9,10 (point de fusion: 210—212°C). Le rendement est de 86,7% sans prendre en compte la fraction de produit restant en solution dans l'acide acétique.

**Revendications**

1. Procédé pour la préparation du tétrahydro - 1,2,3,4 anthracène - diol - 9,10 caractérisé en ce que l'on effectue l'hydrogénation catalytique en phase liquide de la tétrahydro - 1,4,4a,9a anthraquinone en hexahydro - 1,2,3,4,4a,9a anthracène - dione - 9,10 en présence d'un catalyseur à base de nickel ou de métal précieux, à une température comprise entre 20 et 200°C et effectue l'isomérisation de l'hexahydro - 1,2,3,4,4a,9a anthracène - dione - 9,10 en tétrahydro - 1,2,3,4 anthracène - diol - 9,10 en présence d'un acide en l'absence d'oxygène.

2. Procédé tel que revendiqué sous 1) dans lequel l'hexahydro - 1,2,3,4,4a,9a anthracène - dione - 9,10 est séparée du tétrahydro - 1,2,3,4 anthracène - diol - 9,10 et soumise ensuite à l'isomérisation en présence d'un acide.

3. Procédé tel que revendiqué sous 1) dans lequel le mélange d'hexahydro - 1,2,3,4,4a,9a anthracène - dione - 9,10 et de tétrahydro - 1,2,3,4 anthracène - diol - 9,10 est traité par un acide.

4. Procédé tel que revendiqué sous 1) dans lequel l'hydrogénation et l'isomérisation sont effectuées en une seule étape, l'acide étant présent dans le milieu d'hydrogénation.

5. Procédé tel que revendiqué sous 1) dans lequel le solvant est dépourvu de groupement fonctionnel hydrogénable.

6. Procédé tel que revendiqué sous chacune des revendications 1 et 5 dans lequel la température d'hydrogénation est comprise de préférence entre 60 et 130°C.

7. Procédé tel que revendiqué sous chacune des revendications 1, 5 et 6 dans lequel l'hydrogénation est effectuée sous pression, de préférence entre 10 et 50 bars.

8. Procédé tel que revendiqué sous chacune des revendications 1, 2, 3, 4 où l'acide utilisé pour l'isomérisation est un acide minéral ou organique.

**Patentansprüche**

1. Verfahren zur Herstellung von 1.2.3.4 - Tetrahydro - anthracen - 9.10 - diol, dadurch

**0 006 791**

gekennzeichnet, daß man die katalytische Hydrierung in flüssiger Phase des 1.4.4a.9a - Tetrahydroanthrachinons in 1.2.3.4.4a.9a - Hexahydroanthracen - 9.10 - dion in Anwesenheit eines Katalysators auf der Basis von Nickel oder eines Edelmetalls bei einer Temparatur zwischen 20 und 200°C und die Isomerisierung des 1.2.3.4.4a.9a - Hexahydroanthracen - 9.10 - dions zu 1.2.3.4 - Tetrahydroanthracen - 9.10 - diol in Gegenwart einer Säure und Abwesenheit von Sauerstoff durchführt.

2. Verfahren nach Anspruch 1, dadurch gekennzeichnet, daß 1.2.3.4.4a.9a - Hexahydroanthracen - 9.10 - dion von 1.2.3.4 - Tetrahydroanthracen - 9.10 - diol abgetrennt und anschließend der Isomerisierung in Anwesenheit einer Säure unterworfen wird.

3. Verfahren nach Anspruch 1, dadurch gekennzeichnet, daß das Gemisch aus 1.2.3.4.4a.9a - Hexahydroanthracen - 9.10 - dion und 1.2.3.4 - Tetrahydroanthracen - 9.10 - diol mit einer Säure behandelt wird.

4. Verfahren nach Anspruch 1, dadurch gekennzeichnet, daß die Hydrierung und Isomerisierung in einer Stufe durchgeführt werden, wobei im Hydrierungsmilieu Säure anwesend ist.

5. Verfahren nach Anspruch 1, dadurch gekennzeichnet, daß das Lösungsmittel von hydrierbaren funktionellen Gruppen frei ist.

6. Verfahren nach einem der Ansprüche 1 und 5, dadurch gekennzeichnet, daß die Hydrierungstemperatur vorzugsweise zwischen 60 und 130°C liegt.

7. Verfahren nach einem der Ansprüche 1, 5 und 6, dadurch gekennzeichnet, daß die Hydrierung unter Druck, vorzugsweise zwischen 90 und 50 bar ausgeführt wird.

8. Verfahren nach einem der Ansprüche 1, 2, 3 und 4, dadurch gekennzeichnet, daß die für die Isomerisierung verwendete Säure eine Mineralsäure oder eine organische Säure ist.

## Claims

1. Process for the preparation of 1,2,3,4 - tetrahydro - anthracene - 9,10 - diol, characterised in that the liquid phase catalytic hydrogenation of 1,4,4a,9a - tetrahydro-anthraquinone into 1,2,3,4,4a,9a - hexahydro - anthracene - 9,10 - dione is effected in the presence of a catalyst based on nickel or a precious metal, at a temperature between 20 and 200°C and that isomerisation of 1,2,3,4,4a,9a - hexahydro-anthracene - 9,10 - dione into 1,2,3,4 - tetrahydro-anthracene - 9,10 - diol is effected in the presence of an acid and the absence of oxygen.

2. Process such as that claimed under 1), in which 1,2,3,4,4a,9a - hexahydro-anthracene - 9,10 - dione is separated from 1,2,3,4 - tetrahydro-anthracene - 9,10 - diol and then subjected to isomerisation in the presence of an acid.

3. Process such as that claimed under 1), in which the mixture of 1,2,3,4,4a,9a - hexahydro-anchracene - 9,10 - dione and 1,2,3,4 - tetrahydro-anthracene - 9,10 - diol is treated with an acid.

4. Process such as that claimed under 1), in which the hydrogenation and the isomerisation are effected in a single stage, the acid being present in the hydrogenation medium.

5. Process such as that claimed under 1), in which the solvent is free from a hydrogenatable functional group.

6. Process such as that claimed under each of claims 1 and 5, in which the hydrogenation temperature is preferably between 60 and 130°C.

7. Process such as that claimed under each of claims 1, 5 and 6, in which hydrogenation is effected under pressure, preferably between 10 and 50 bars.

8. Process such as that claimed under each of claims 1, 2, 3, 4, in which the acid used for isomerisation is a mineral or organic acid.

4